# FASCICULE DE BREVET EUROPEEN

(11) **EP 0 869 969 B1**
(45) Date de publication et mention de la délivrance du brevet: **26.03.2003**
(21) Numéro de dépôt: 96939131.7
(22) Date de dépôt: 15.11.1996
(51) Int. Cl.: C07K 7/06, C07K 5/08, C07K 5/10, A61K 38/04

(54) **CONJUGUES PEPTIDIQUES, LEUR UTILISATION A TITRE DE MEDICAMENT ET COMPOSITIONS LES CONTENANT**
PEPTIDKONJUGATE, DEREN VERWENDUNG ALS HEILMITTEL UND ZUSAMMENSeTZUNGen, DIEse ENTHALTENd
PEPTIDE CONJUGATES, USE THEREOF AS A DRUG, AND COMPOSITIONS CONTAINING them

(30) Priorité: 15.11.1995 FR 9513543
(43) Date de publication de la demande: 14.10.1998
(73) Titulaire: INSTITUT EUROPEEN DE BIOLOGIE CELLULAIRE, F-31520 Ramonville-Saint-Agne (FR)
(72) Inventeur: DUSSOURD D'HINTERLAND, Lucien, F-31400 Toulouse (FR); PINEL, Anne-Marie, F-34280 La Grande-Motte (FR)
(74) Mandataire: Warcoin, Jacques
(86) Numéro de dépôt international: FR9601811
(87) Numéro de publication internationale: WO97018235

(56) Documents cités:
- WO-A-91/07431
- WO-A-91/12267
- DE-A- 4 127 790

## Description

Sous l'influence de processus physiologiques internes "peptides neurotransmetteurs" ou externes "radiations ionisantes et ultraviolettes", les cellules de l'épiderme, en particulier les kératinocytes germinatifs sécrètent des facteurs de croissance et de coopération cellulaire, dont le rôle est de stimuler les synthèses cellulaires des molécules du cytosquelette et d'activer les interactions cellulaires.

Ces propriétés physiologiques se traduisent par une activation des métabolismes cellulaires indispensables à la régénération des tissus conjonctifs du derme et de l'épiderme et des processus de cicatrisation.

La présente Invention a pour objet la réalisation de molécules peptidiques, dérivés homologues des séquences peptidiques actives des facteurs de croissance et de coopération cellulaires, sécrétés naturellement par les kératinocytes.

Parmi les dérivés peptidiques faisant l'objet de la présente invention figurent notamment des dérivés peptidiques et métallo-peptidiques dont les activités pharmacologiques sont orientées :
- d'une part, vers les synthèses cellulaires des molécules composant le cytosquelette telles que les collagènes de type I et III, les glycosaminoglycanes, les fibronectines,
- d'autre part, vers la synthèse des molécules dont le rôle est de stimuler et d'activer les phénomènes de coopération et d'interaction cellulaire "endothélines, intégrines".

Ces activités étant complémentaires et indispensables à l'expression au niveau du derme et de l'épiderme, des processus de restructuration des tissus, cicatrisation, angiogénèse, mélanogénèse, etc...

Un des objets de la présente invention est l'utilisation des dérivés peptidiques et métallo-peptidiques précédents, pour le traitement en application par voie topique, de la cicatrisation des plaies chroniques, telles que les lésions ulcéreuses du diabétique, les ulcères variqueux, la cicatrisation esthétique des plaies chirurgicales, le traitement préventif et curatif des vergetures et de leurs complications.

Dans les indications précédentes, les dérivés peptidiques peuvent être utilisés en thérapeutique humaine et vétérinaire sous forme de "pommades, gels, solutions ou de spray".

Une des applications privilégiée de l'invention est l'utilisation des dérivés peptidiques dans les différents domaines de la cosmétologie, pour le traitement préventif et curatif des rides, du visage, du cou, et des mains, sous forme de lotions, gels, laits, crèmes, ou de spray, en application locale.

Une autre application des dérivés peptidiques de l'invention est leur utilisation comme agent d'activation et de potentialisation des mécanismes physiologiques de coopérations cellulaires.

Les dérivés peptidiques peuvent être utilisés seuls ou de préférence en association, avec des biomolécules ou composés naturels ou leurs dérivés de synthèse, dont l'activité biologique spécifique, ne peut s'exprimer pleinement, que par la collaboration entre deux systèmes cellulaires complémentaires dont ils stimulent ou induisent qu'un seul élément.

C'est le cas, en particulier, des hormones mélanotropes qui induisent la mélanogénèse dans les cellules mélanocytaires et dont l'expression au niveau de la surface de l'épiderme humain (race blanche) ne peut s'exprimer pleinement que par l'intime coopération entre les cellules mélanocytaires et les kératinocytes, formant ainsi une véritable unité fonctionnelle dénommée "Epidermal Melanin Unit".

Les mélanocytes activés par l'αMSH et ses dérivés sécrètent la mélanine, qui est transférée et dispersée, dans les kératinocytes, sous l'influence des facteurs peptidiques de coopération cellulaire et de leurs dérivés homologues.

Une des applications de la présente invention est l'utilisation des dérivés peptidiques, précédemment décrits, dans les différents domaines de la dermatologie et de la cosmétologie.

Les dérivés peptidiques peuvent être utilisés seuls ou en association avec d'autres molécules actives, tels que les dérivés homologues de synthèse de l'aMSH, ou autres substances, sous la forme de crèmes, laits, lotions, solutions ou sprays, en applications topiques (locales), pour l'accélération des processus de mélanogénèse de l'épiderme, accélération de la mélanisation de la peau par obtention d'un bronzage naturel et d'une protection totale contre les radiations solaires (UV).

La présente invention concerne des conjugués comportant une séquence peptidique comprenant au moins trois acides aminés choisis parmi Gly-His-Lys et Glu-His-Lys,
dans laquelle "Lys" représente la Lysine ou un dérivé halogéné de la Lysine, ou un dérivé méthylé de la Lysine, notamment Méthyl-Lysine et Dihydrobromo-Méthyl-Lysine, les acides aminés pouvant être sous la forme D, L ou DL.

Ces acides aminés étant sous leur forme naturelle ou non, ladite séquence étant utilisée sous forme peptidique, ou conjuguée chimiquement ou physiquement avec au moins un composé sélectionné parmi
1) les acides monocarboxyliques de formule générale

   HOOC-R (I)

   dans laquelle R représente un radical aliphatique en C₁ à C₂₀ droit ou ramifié, substitué ou non, notamment un radical alkyl, alkényl ou alkynyl pouvant comporter une ou plusieurs insaturations et pouvant être substitué par un ou plusieurs radicaux choisis dans le groupe comprenant : NH₂, OH, oxo ou un radical cyclique non aromatique comportant de 5 à 6 atomes dans le cycle dont 1 ou 2 pouvant être différents du carbone, en particulier, S, O ou N, lesdits cycles pouvant être substitués par des radicaux alkyl en C₁ à C₃, notamment méthyl,
   ainsi que les dérivés alcool ou aldéhyde ou amide des acides de formule 1 ; à la condition que si la séquence peptidique comprend uniquement Gly-His-Lys, elle n'est pas conjuguée à un seul résidu d'acide palmilique ; Parmi les cycles pouvant être substitués, il faut citer les cycles 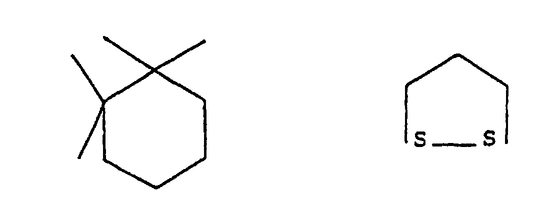 correspondant à des dérivés de l'acide rétinoïque et de l'acide lipoïque.
   Dans certains cas, la chaine aliphatique peut consister en une chaîne polyalkényl.
   Parmi les composés de formule I il faut citer de préférence les composés de formule :

   R₂-CH=CH-COOH

   de configuration cis ou trans dans laquelle R₂ est un radical alkyl en C₆ à C₁₆, comportant éventuellement un ou plusieurs substituants -NH₂, OH ou oxo.
2) les acides dicarboxyliques de formule générale

   HOOC-R₁-COOH (II)
dans laquelle R₁ représente un radical aliphatique divalent notamment en C₃ à C₁₀, droit ou ramifié, substitué ou non, notamment un radical alkyl, alkylène, alkényléne ou alkynylène, pouvant comporter une ou plusieurs insaturations et pouvant être substitué par un ou plusieurs radicaux NH₂. OH, oxo ou un radical alkyl en C₁ à C₃.

Dans la formule générale I, il faut citer plus particulièrement les composés dans lesquels R représente :
- un radical monoinsaturé de configuration cis ou trans, de formule générale

   R₂-CH=CH-
dans laquelle R₂ représente un radical aliphatique notamment alkyl, linéaire ou ramifié comprenant au moins 6 atomes de carbone, de préférence 6 à 16 atomes de carbone, substitué par un groupe amino, hydroxy ou oxo ;
- un radical alkyl linéaire ou ramifié en C₁-C₂₀ éventuellement susbtitué par un ou plusieurs substituants choisi parmi ; amino, hydroxy, oxo, thiol, imino, cycloalkyl en C₄-C₇, éventuellement substitué et si au moins 2 substituants sont présents, ils peuvent former ensemble une liaison, notamment un pont disulfure.

Les peptides conjugués selon l'invention sont liés sous forme de sels, d'esters, ou d'amides à des acides possédant des fonctions métaboliques essentielles dans l'activation du cycle tricarboxylique de Krebs.

Les acides de formule générale (I) ou (II) sont plus particulièrement choisis parmi les acides carboxyliques pour lesquels R₁ représente un alkylène en C₄-C₈ substitué ou non, en particulier, les acides acétiques, adipiques, α-amino-adipiques et dérivés, l'acide DL lipoïque et dérivés, l'acide dihydrolipoïque et son dérivé N-Lipoyl-Lysine, l'acide pimélique et dérivés, l'acide sébacique et dérivés.

Les acides gras pour lesquels R représente un reste alkylène linéaire ou ramifié, sont plus particulièrement les acides hydroxydécénoïques et décénoïliques, les acides rétinoïques et ses dérivés, le Rétinal et le Rétinol, l'acide myristique et ses dérivés, et l'acide palmitique, sous forme de sels d'esters, ou d'amides.

Des composés spécialement adaptés à l'obtention de conjugués selon l'invention sont choisis parmi l'acide acétique et ses dérivés, l'acide α-DL-Lipoïque et ses dérivés, l'acide dihydrolipoïque, la N-Lipoyl-Lysine, l'acide adipique, l'acide α-amino-adipique, l'acide pimélique, l'acide sébacique et ses dérivés, l'acide trans-10-hydroxy-Δ2-décénoïque et l'acide trans-oxo-9-decene-2-oïque, l'acide rétinoïque et ses dérivés, le rétinol, et le rétinal, l'acide myristique et l'acide palmitique.

Plus spécifiquement, la présente invention concerne des peptides inducteurs des synthèses endocellulaires des collagènes de type I et III et des glycosaminoglycanes, à haute activité cicatrisante et métabolique, ainsi que des molécules d'induction et de stimulation des processus de coopération cellulaire.

Les conjugués peptidiques selon l'invention comprennent au moins une des séquences peptidiques suivantes :

Gly-His-Lys

Glu-His-Lys

dans laquelle Lys représente la lysine ou un dérivé halogéné de la lysine, ou un dérivé méthylé de la lysine, notamment Méthyl-Lysine et Dihydrobromo-Méthyl-Lysine, les acides aminés pouvant être sous la forme D, L ou DL.

Les conjugués peptidiques répondent à l'une des formules générales suivantes :

A-X-Gly-His-Lys-Y (III),

ou

A-X-Glu-His-Lys-Y (IV)

dans laquelle
. A est un composé de formule générale I ou II ou le radical correspondant à ce composé
. X représente une chaine de 1 à 3 résidus Lys, éventuellement méthylée, en particulier à l'extrémité N terminale, OH, NH₂ ou une liaison
. Y représente OH ou NH₂,
les acides aminés étant sous forme D, L ou DL
A est de préférence l'acide acétique, l'acide adipique, l'acide α-amino-adipique, l'acide DL lipoïque, l'acide dihydrolipoïque, la N-Lipoyl-Lysine, l'acide pimélique, l'acide sébacique, l'acide trans-oxo-9-décéne-2 oïque et l'acide trans-hydroxy-10-décéne-2-oïque.

La présente invention concerne particulièrement les conjugués peptidiques suivants :
1 - A-MeLys-Lys-Lys-Gly-His-Lys-NH₂
2 - A-MeLys-Lys-Gly-His-Lys-NH₂
3 - A-MeLys-Gly-His-Lys-NH₂
4 - A-MeLys-Lys-Lys-Gly-His-Lys-OH
5 - A-MeLys-Lys-Gly-His-Lys-OH
6 - A-MeLys-Gly-His-Lys-OH
7- A-Lys-Lys-Gly-His-Lys-NH₂
8- A-Lys-Gly-His-Lys-NH₂
9 - A-Lys-Lys-Gly-His-Lys-OH
10- A-Lys-Gly-His-Lys-OH
11- A-Lys-Lys-Glu-His-Lys-NH₂
12 - A-Lys-Glu-His-Lys-NH₂
13 - A-Glu-His-Lys-NH2
14- A-Lys-Lys-Glu-His-Lys-OH
15 - A-Lys-Glu-His-Lys-OH
16- A-Glu-His-Lys-OH

A étant défini comme précédemment, ainsi que les dérivés de ces molécules sous forme de sels, d'esters, ou d'amides.

Les séquences d'acides aminés, mentionnés ci-dessus peuvent être des séquences d'acides aminés naturels ou non naturels.

Les conjugaisons selon la présente invention peuvent être effectués en faisant réagir la fonction amine ou acide de l'acide aminé avec une fonction amine ou acide de l'acide A, ou méme il est possible de mettre à profit la présence d'une fonction hydroxy sur l'acide.

La présente invention concerne l'ensemble de ces conjugaisons ainsi que les conjugués non fonctionnels.

Les conjugués peptidiques précédemment décrits dans l'invention, peuvent être utilisés sous leur forme peptidique, ou liés avec un métal sous forme de complexes équimoléculaires.

Le métal utilisé pour la réalisation des complexes métallo-peptidiques de l'invention est de préférence un métal divalent notamment le Zinc (Zn), qui peut être couplé sous forme de sel, avec le groupement carboxylique de l'amino-acide terminal, la lysine (Lys).

De même, dans certains cas, il est possible que certains amino-acides comportent par exemple des fonctions de glycosylation.

Il doit être entendu que la présente invention concerne également l'ensemble de ces formes.

Selon un autre aspect, l'invention concerne des compositions galéniques contenant au moins un conjugué peptidique tel que défini précédemment, avec des excipients cosmétologiquement et/ou pharmaceutiquement acceptables.

La présente invention concerne plus particulièrement l'utilisation à titre de médicament d'un, ou plusieurs des conjugués et dérivés peptidiques décrits précédemment selon l'invention.

La présente invention concerne également des préparations cosmétiques, contenant un, ou plusieurs conjugués et dérivés peptidiques décrits précédemment selon l'invention.

La présente invention concerne également des compositions galéniques, pharmaceutiques et cosmétologiques, comprenant au moins un des composés tels que défini précédemment.

Un des objets de la présente invention est l'utilisation par voie topique ou injectable des conjugués peptidiques et de leurs dérivés, composition ou association avec des principes actifs connus.

Ces conjugués peptidiques, leurs dérivés, composition ou association, peuvent être présentés sous forme d'ampoules injectables lyophilisées, et sous forme de crème, gels, laits, lotions ou sprays, et comporter des excipients connus.

Les conjugués peptidiques de l'invention, leurs dérivés, compositions, ou associations avec des principes actifs connus, sont utiles pour le traitement et la cicatrisation des plaies et lésions chroniques du diabétique, les ulcères variqueux, la cicatrisation des plaies chirurgicales, le traitement préventif et curatif des vergetures.

Le conjugé pourra notamment être associé à au moins une substance choisie parmi les antiseptiques, les antibiotiques à large spectre antimicrobien, ou les antifongiques à large spectre d'activité, pour la préparation d'une composition destinée au traitement par voie topique et à la cicatrisation des plaies infectées.

Selon un autre aspect, le conjugué peptidique peut être présenté sous forme d'associations ou de composés, avec des molécules connues pour leurs activités anticoagulantes et phlébotoniques par voie topique, destinées au traitement préventif et curatif, des vergetures, des insuffisances veineuses (jambes lourdes), de la couperose, en applications locales, sous forme de crèmes de gels, de laits ou de sprays.

Une des applications préférentielle de l'invention est l'utilisation des conjugués peptidiques, comme adjuvants, activateurs ou cofacteurs, en association avec des molécules choisies parmi l' αMSH ou ses dérivés homologues, l'ACTH ou la propiomélanocortine et leurs analogues dans des compositions destinées au traitement préventif et curatif des troubles de la mélanogénèse, à la stimulation de la mélanogénèse épidermique, en vue de l'obtention d'un bronzage naturel rapide et d'une protection totale contre les radiations solaires ultraviolettes (DEM).

Une autre des applications préférentielles de l'invention est l'utilisation des conjugués peptidiques dans les différentes formulations cosmétologiques pour le traitement préventif et curatif des rides, du visage, du cou et des mains, sous forme d'émulsions, de crèmes, de laits, de lotions, de gels, ou de sprays en application locale.

Les exemples qui suivent sont destinés à illustrer l'invention sans aucunement en limiter la portée.

Dans ces exemples on se référera aux Figures suivantes :
Figure 1 : Intensité de fluorescence de fibroblastes marqués à la rhodamine : mise en évidence du collagène de type I.
Figures 2 et 3 : Mise en évidence par mesure de l'intensité de coloration de la synthèse de collagène par des cellules traitées ou non par le conjugué n° 7.

### Exemple n° 1 - Synthèse du conjugué 7

A-Lys-Lys-Gly-His-Lys-NH₂

A = Acide Adipique = COOH-(CH₂)₄-COOH

La synthèse a été réalisée par la méthode de Merrifield adaptée à la structure du conjugué selon les étapes suivantes :

Z-Gly-His-Lys-NH₂ :

A une solution dans la diméthylformamide (10 mL) contenant Z-Gly-OH (2.08 g, 10 mmoles), BOP (4.42 g, 10 mmoles), de la diisopropyléthylamine (DIEA, 22 mmoles) est additionné sous agitation et à 0° C, le chlorhydrate de H-His-Lys-NH₂ (2.1 g, 11 mmoles).

Après 6 heures d'agitation à la température ambiante, le solvant de la réaction est évaporé sous vide à une température inférieure à 40° C.

Le résidu est dissout dans de l'acétate d'éthyle (200 mL).

La phase organique est lavée plusieurs fois avec une solution saturée de bicarbonate de sodium (2 x 50 mL), à l'eau (2 x 50 mL), avec une solution saturée de KHSO₄ (2 x 50 mL), à l'eau (2 x 50 mL).

La phase organique est séchée sur sulfate de sodium et concentrée sous vide pour conduire à une mousse blanche (3.1 g. 91%) homogène en chromatographie sur gel de silice.

Z-Lys(Boc)-Gly-His-Lys-NH₂

Le composé Z-Gly-His-Lys-NH₂ (3.45 g, 10 mmoles) est dissout dans l'éthanol 95 (100 mL) contenant 20 mmoles d'acide chlorhydrique.

Le mélange réactionnel est hydrogéné pendant 5 heures.

Le catalyseur est filtré et le solvant concentré sous vide à une températurè inférieure à 40° C.

Le résidu restant est trituré plusieurs fois avec de l'éther, décanté. Il est séché sous vide en présence de KOH. Ce résidu est dissout dans la diméthylformamide (10 mL) en présence de Z-Lys(Boc)-OH, (3,81 g, 10 mmoles), BOP (4.42 g, 10 mmoles), de la diisopropyléthylamine (DIEA, 22 mmoles).

Le mélange réactionnel est agité 30 min à 0° C, puis 4 heures à la température ambiante.

Le solvant de la réaction est évaporé sous vide à une température inférieure à 40° C.

Le résidu est dissout dans de l'acétate d'éthyle (200 mL).

La phase organique est lavée plusieurs fois avec une solution saturée de bicarbonate de sodium (2 x 50 mL), à l'eau (2 x 50 mL), avec une solution saturée de KHSO4 (2 x 50 mL), à l'eau (2 x 50 mL). La phase organique est séchée sur sulfate de sodium et concentrée sous vide pour conduire à une mousse blanche (4.7 g, 82%) homogène en chromatographie sur gel de silice.

Z-Lys-(Boc)-Lys-(Boc)-Gly-His-Lys-NH₂

Le composé ZLys(Boc)-Gly-His-Lys-NH22 (5.7 g, 10 mmoles) est dissout dans l'éthanol 95 (150 mL) contenant 10 mmoles d'acide chlorhydrique.

Le mélange réactionnel est hydrogéné pendant 5 heures.

Le catalyseur est filtré et le solvant concentré sous vide à une température inférieure à 40° C.

Le résidu restant est trituré plusieurs fois avec de l'éther, décanté. Il est séché sous vide en présence de KOH. Ce résidu est dissout dans la diméthylformamide (10 mL) en présence de Z-Lys(Boc)-OH, (3,81 g, 10 mmoles), BOP (4.42 g, 10 mmoles), de la diisopropyléthylamine (DIEA, 22 mmoles).

Le mélange réactionnel est agité 30 min à 0° C, puis 6 heures à la température ambiante.

Le solvant de la réaction est évaporé sous vide à une température inférieure à 40° C.

Le résidu est dissout dans de l'acétate d'éthyle (300 mL).

La phase organique est lavée plusieurs fois avec une solution saturée de bicarbonate de sodium (2 x 50 mL), à l'eau (2 x 50 mL), avec une solution saturée de KHSO4 (2 x 50 mL), à l'eau (2 x 50 mL). La phase organique est séchée sur sulfate de sodium et concentrée sous vide pour conduire à une mousse blanche (6.5 g, 81%) homogène en chromatographie sur gel de silice.

A-Lys-Lys-Gly-His-Lys-NH₂

Le composé ZLys(Boc)-Lys(Boc)-Gly-His-Lys-NH₂ (8 g, 10 mmoles) est dissout dans l'éthanol 95 (200 mL) contenant 20 mmoles d'acide chlorhydrique.

Le mélange réactionnel est hydrogéné pendant 5 heures.

Le catalyseur est filtré et le solvant concentré sous vide à une température inférieure à 40° C.

Le résidu restant est trituré plusieurs fois avec de l'éther décanté. Il est séché sous vide en présence de KOH. Ce résidu est dissout dans la diméthylformamide (10 mL) en présence d'acide adipique (2.1 g, 10 mmoles), BOP (4.42 g, 10 mmoles), de la diisopropyléthylamine (DIEA, 22 mmoles).

Le mélange réactionnel est agité 15 min à O° C, puis 5 heures à la température ambiante.

Le solvant de la réaction est évaporé sous vide à une température inférieure à 40° C.

Le résidu est dissout dans de l'acétate d'éthyle (200 mL).

La phase organique est lavée plusieurs fois avec une solution saturée de bicarbonate de sodium (2 x 50 mL), à l'eau (2 x 50 mL), avec une solution saturée de KHSO4 (2 x 50 mL), à l'eau (2 x 50 mL). La phase organique est séchée sur sulfate de sodium et concentrée sous vide pour conduire à une poudre blanche (7.5 g, 76%) homogène en chromatographie sur gel de silice.

Ce composé est dissout dans l'acide trifluoroacétique (50 mL).

Après 30 min, le solvant est concentré sous vide à une température inférieure à 40° C et le résidu trituré plusieurs fois avec de l'éther anhydre.

La poudre blanche obtenue (6.9 g, 88%) est séchée sous vide.

Adipoyl-Lys-Lys-Gly-His-Lys-NH₂

### Exemple n° 2 - Synthèse du conjugué 8

A-Lys-Gly-His-Lys-NH₂

A = Trans-10-Hydroxy-déc2-enoïc-acide

La synthèse a été réalisée par la méthode de Merrifield adaptée selon les étapes suivantes et la méthodologie décrite à l'exemple n°1 :
1 - Z-Gly-OH + H-His-Lys-NH₂
2 - Z-Gly-His-Lys-NH₂
3 - Z-Lys-(BOC)-Gly-His-Lys-NH₂
4 - A-BOP, DIEA, DMF
5 - Decenoyl-Lys-Gly-His-Lys-NH₂

Préparation du complexe métallo-peptidique avec le Zinc.

On utilise le sel de Zinc sous forme de Zncl2.

Le conjugué peptidique précédemment obtenu est mis en solution à pH 5,6 dans l'eau distillée dans les proportions suivantes :
1 molécule du conjugué n° 8 dans une solution de Zncl2 à 10%.

Après chauffage à 20° C pendant 10 mn, le complexe est purifié sur colonne de Biogel P-2 pour éliminer l'excès de chlorure de Zinc non fixé sur le peptide, et élué avec de l'eau distillée.

Après élution, on obtient une solution contenant 0,5% ce Zncl2 pour une molécule peptidique.

La solution est ensuite congelée à basse température "-60° C" et lyophilisée.

On obtient le complexe métallo-peptidique sous forme de poudre blanche dont la formule est la suivante :

HO(CH₂)₇-CH=CH-CO-Lys-Gly-His-Lys-Zn

ou

(Trans-10-hydroxy-dec2-enoïc-Lys-Gly-His-Lys-Zn)

dont les caractères analytiques sont les suivants :

| | |
|---|---|
| Caractères organoleptiques : | Lyophilisat blanc soluble dans l'eau. |
| | |
| Pureté HPLC : | 9596. |
| | |
| Spectrométrie de masse "hors Zinc" | PM 636.4-6363.6 |
| | |
| Composition en acides aminés (% hors Zinc) | |
| Lysine | 42.95% |
| Histidine | 21,55% |
| Glycine | 8,97% |

### Exemple 3 - Etude du pouvoir cicatrisant du conjugué peptidique n° 7 chez l'animal "rat"

### But de l'étude

Déterminer le pouvoir cicatrisant du peptide n° 7 administré chez le rat-OFA par applications topiques aux doses de 0,75 µg, 7,5 µg et 75 µg par animal pendant cinq jours.

### Matériel et méthode

### I - Animaux : rats de souche OFA, OF1, IFFA-CREDO, pesant de 250 à 300 g.

### II - Produits à tester

Conjugué peptidique n° 7, correspondant la formule :

Adipoyl-Lys-Lys-Gly-His-Lys-NH₂.

Mis en solution dans le propylène glycol aux doses de 1,5 µg/ml, 15 µg/ml, 150 µg/ml.

### III - Méthode

Après anesthésie générale à l'éther, une incision de 5 cm de long a été pratiquée sur la flanc droit des animaux.

Une suture en fil trinyl 2/0 a été effectuée à raison d'un point tous les 0,5 cm.

Les animaux traités comme les témoins ont subi la même intervention dans les mêmes conditions.

Le traitement a débuté le lendemain de l'intervention sur les lots d'animaux traités aux doses de 0,5 ml de solution peptidique sur chaque incision.

Soit 0,75 µg, 7,5 µg et 75 µg de peptide par animal et par application pendant la durée du traitement, soit 5 jours.

Les animaux ont été mis en observation pendant 9 jours, ils ont ensuite été sacrifiés par une dose létale de penthiobarbital.

Les zones de peau en cours de cicatrisation ont été prélevées et soumises aux "tests" d'étirement au dynamomètre, pour mesurer la résistance de la cicatrice à l'étirement.

### Analyse statistique des résultats

Une analyse statistique a été effectuée sur les valeurs de résistance obtenue au dynamomètre.
Une analyse de variance et un test de "T" student a été pratiqué sur les valeurs individuelles, afin de définir le seuil de signification des résultats.

### Résultats

Le peptide n°7, administré aux doses de 0,75 µg, 7,5 µg et 75 µg, par jour et pendant 5 jours consécutifs, aux animaux en expérimentation, augmente de façon hautement significative, la cicatrisation des plaies chez les animaux traites par rapport aux témoins, aux doses de :
0,75 µg l'augmentation est de 14,5%,
7,5 µg l'augmentation est de 30,9%,
75 µg l'augmentation est de 59,6%.

On observe en particulier une cicatrisation totale chez les animaux traités, à partir du 3ème jour, alors que les témoins ne présentent pas de signe visible de cicatrisation.

L'aspect des cicatrisations au 9ème jour présente chez les animaux traités, une cicatrisation parfaitement plate, avec absence de bourrelets cicatriciels.

**Tableau n° 1**

| **Animaux traités par 0,75 µg/jour/5 jours par peptide formule n° 7** **Résistance des cicatrices à l'étirement "dynamométrie"** | | | |
|---|---|---|---|
| Groupe Témoin | | Groupe Traité | |
| Animaux | Dynes | Animaux | Dynes |
| 894923 | 26,2 | 894918 | 28,5 |
| 894924 | 23,7 | 894919 | 26,1 |
| 894925 | 22,5 | 894920 | 29,3 |
| 894926 | 25,4 | 894921 | 27,7 |
| 894927 | 24,8 | 894922 | 28,6 |
| Moyenne | 24,5+/-1,5 | Moyenne | 28,0+/-1,2 |

**Tableau n° 2**

| **Animaux traités par 7,5 µg/jour/5 jours par peptide formule n° 7** | | | |
|---|---|---|---|
| Groupe Témoin | | Groupe Traité | |
| Animaux | Dynes | Animaux | Dynes |
| 894923 | 26,2 | 894928 | 32,6 |
| 894924 | 23,7 | 894929 | 30,4 |
| 894925 | 22,5 | 894930 | 33,1 |
| 894926 | 25,4 | 894931 | 32,3 |
| 894927 | 24,8 | 894932 | 31,8 |
| Moyenne | 24,5 +/- 1,5 | Moyenne | 32,0 +/- 1,0 |

**Tableau n° 3**

| **Animaux traités par 7,5 µg/jour/5 jours par pcptidc formule n° 7** | | | |
|---|---|---|---|
| Groupe Témoin | | Groupe Traité | |
| Animaux | Dynes | Animaux | Dynes |
| 894923 | 26,2 | 894933 | 39,8 |
| 894924 | 23,7 | 894934 | 41,3 |
| 894925 | 22,5 | 894935 | 37,5 |
| 894926 | 25,4 | 894936 | 36,2 |
| 894927 | 24,8 | 894937 | 40,7 |
| Moyenne | 24,5 +/- 1,5 | Moyenne | 39,1+/-2,2 |

### Conclusions

Les résultats obtenus au cours de cette étude permettent de conclure que le peptide n° 7 possède une action cicatrisante hautement significative.

Pour les doses de 75 µg par jour pendant 5 jours la différence entre le groupe traité et le groupe témoin est de 59,6%.

### Exemple 4 - Mise en évidence de l'activité du conjugué n° 7 sur la synthèse de collagène de type I par des fibroblastes en culture

La mise en évidence de l'influence du conjugué n' 7 sur la synthèse de collagène de type I par des cellules en culture a été réalisée sur deux types cellulaires :
- des fibroblastes humains de plastie mammaire au 3ème passage, obtenus dans notre laboratoire de culture cellulaire à l'IEB,
- des MRC5, fibroblastes de poumon embryonnaire humain non transformés au passage p38, obtenus à p 25-30 chez ICN FLOW.

Les cellules sont stimulées ou non avec le conjugué n° 7, puis elles sont observées au microscope pholoniquc à fluorescence après révélation du collagène de type I par une réaction d'immuno-fluorescence secondaire.

### Matériel et méthode

### Culture cellulaire :

Les cellules sont ensemencées à raison de 70 000 cellules par ml pour les fibroblastes de primoculture et 20 000 pour les MRC5.

La culture est réalisée en DMEM 10% SVF dans les tubes de Leigton contenant une lamelle dans le fond du tube, volume final : 1,6 ml.

8 tubes sont prévus par type cellulaire : 3 témoins et 5 concentrations.

### Préparation des produits :

| Préparation d'une solution mère du conjugué n° 7 à 2,34 10-3 M : M1 | | | |
|---|---|---|---|
| **M** | **Vol de M** | **Vol de PBS-BSA** | **Vol M** |
| M2 | 2 ml M1 | 2,68 ml | 10⁻³ M |
| M3 | 0,01 ml M2 | 1 ml | 10⁻⁵ M |
| M4 | 0,01 ml M3 | 1 ml | 10⁻⁷ M |
| M5 | 0,01 ml M4 | 1 ml | 10⁻⁹ M |

Ces différentes solutions mères seront ajoutées au milieu de culture au moment du contact produit.

Les dilutions sont réalisées dans du PBS (Eurobio) - BSA 0,1% (Flucka) puis filtrées sur filtres 0,22 µm (Sartorius).

### Méthodologie

A J0, le milieu est éliminé et remplacé par du milieu frais auquel est ajouté le conjugué n° 7 aux différentes concentrations selon le tableau suivant :

| **C** | **Concentration finale** | **Vol milieu** | **Vol M** |
|---|---|---|---|
| C1 | 10⁻⁴ M | 1,5 ml | 0,15 ml M2 |
| C2 | 10⁻⁵ M | 1,5 ml | 0.015 ml M2 |
| C3 | 10⁻⁷ M | 1,5 ml | 0,015 ml M3 |
| C4 | 10⁻⁹ M | 1,5 ml | 0,015 ml M4 |
| C5 | 10⁻⁷ M | 1,5 ml | 0,015 ml M5 |

Les témoins TO et T ne reçoivent que du milieu.

T1 est traité comme C1.

### Fixation des cellules

Le milieu est élimine ; les tubes sont rincés 3 fois avec du DMEM sans sérum à 37° C.
Les cellules sont fixées 20 mn à 4°C en présence de PFA 2,5% (Prolabo) dans du Hanks (Eurobio).

3 rinçages en DMEM sans sérum sont effectués suivis de 2 lavages 10 mn en PBS sans calcium ni magnésium (Eurobio).

### Marquage des cellules

* Réaction primaire : fixation du premier anticorps de souris anticollagène humain de type 1 dilué au 1/100ème dans du PBS.
   Les lamelles sont sorties des tubes et séchées. La face portant les cellules est repérée et signalée (un angle est cassé pour repère).
   100 ml d'anticorps primaire sont mis au contact des cellules pour les conditions C1 à C5.
   Les lamelles T et T1 restent dans les tubes avec du PBS sans calcium ni magnésium car ce sont des témoins pour le deuxième anticorps.
   Les lamelles sont placées en chambre humide pour éviter la déshydratation à +4° C sous agitation douce durant toute la nuit.
* Réaction secondaire : cette étape révèle la réaction primaire par un anticorps secondaire de chèvre anti-Ig G de souris marqué TRITC : Sigma réf. T-7782 lot 044H4831.
   L'anticorps primaire est éliminé et les lamelles sont lavées 2 fois au PBS pendant 10 mn à température ambiante.
   Puis un lavage en PBS 0,1% BSA est réalisé 10 mn supplémentaires. 100 ml d'anticorps secondaire dilué au 1/120ème sont mis au contact des cellules de toutes les lamelles à température ambiante durant 1 heure.
   L'anticorps est ensuite éliminé et les lamelles sont lavées au PBS 0,196 BSA 10 mn, puis 4 fols 5 mn à l'eau osmosée.
   Une fois séchées les lamelles sont montées côté cellules sur une lame de microscopie contre une goutte de liquide de montage (Sigma). Les bords des lamelles sont lutés au vernis.

### Résultats

Les observations microscopiques sont réalisées sur un microscope droit à fluorescence Zeiss (Axioplan), surmonté d'une caméra 3 CCD Sony (MC-3215 P/M) reliée à un moniteur vidéo Trinitron avec écran couleur Sony. L'ensemble est connecté à une imprimante couleur Sony (UP-7000p). Les observations des lames sont faites avec l'objectif Plan Apochromat x 63 à l'huile.

Les cellules sont d'abord observées en contraste interférentiel et/ou de phase. Quand un champ est sélectionné, l'image est enregistrée, puis sans bouger d'endroit l'observation est réalisée en fluorescence et enregistrée.

Ainsi pour chaque prise de vue nous disposons simultanément à l'écran ou superposées les 2 images mémorisées.

### Conclusions

Comme le montre l'ensemble de ces résultats, le conjugué n° 7 à 10-11 M stimule très significativement la synthèse de collagène de type I par des fibroblastes de primoculture.

La couleur rouge indique la fixation des anticorps secondaires marqués à la rhodamine sur les anticorps anti-collagène de type I. Cette couleur témoigne donc de la présence de collagène de type I synthétisée par les fibroblastes en culture.

La luminosité moyenne du rouge sur l'ensemble de l'image des cellules traitées avec le conjugué n° 7 à 10-11 M est de 54,8 alors que pour les cellules non traitées elle n'est que de 32,78.

Le conjuguée n° 7 stimule la synthèse de collagène de type 1 par les fibroblastes humains de primocultures de manière significative : +67%.

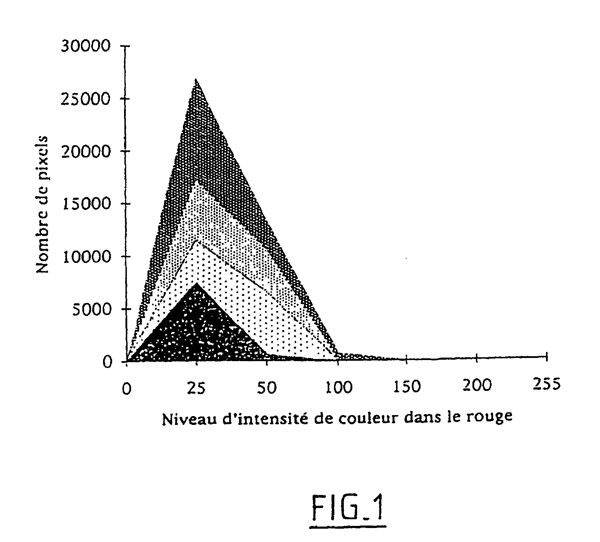

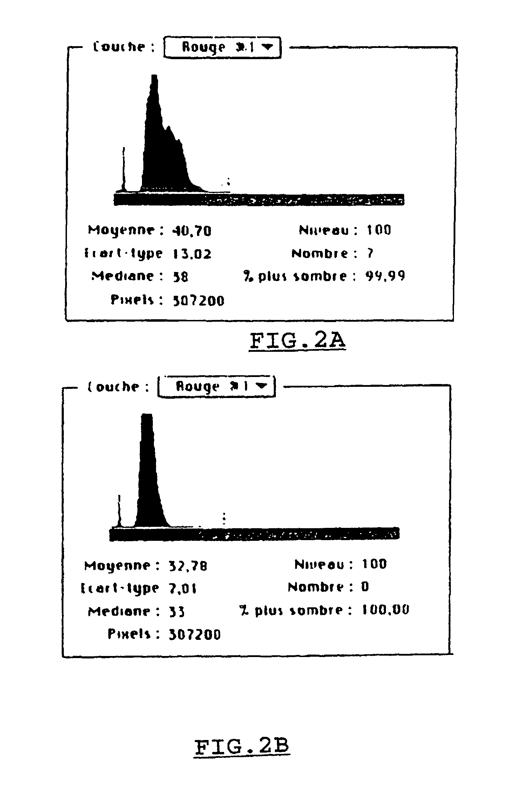

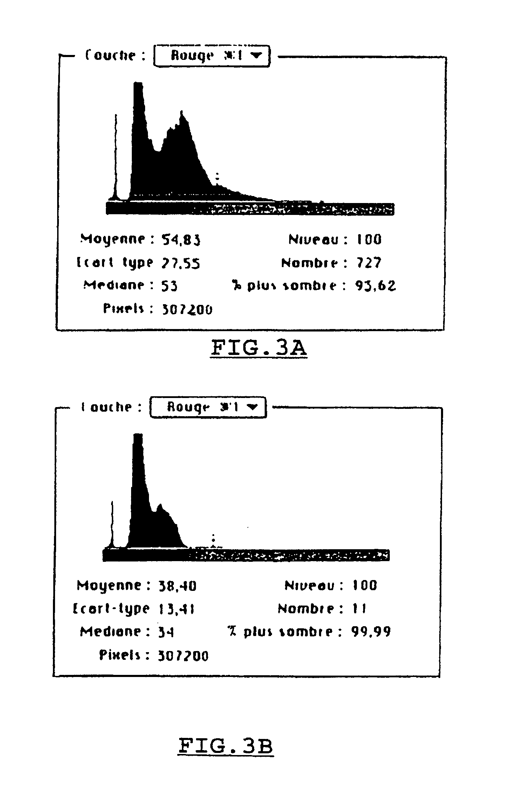

Les résultats non présentés ici, obtenus sur les MRC5 donnent les mêmes tendances.
(Voir figures 1 à 3).

### LEGENDES DES FIGURES

### Figure 1 :

Intensité de fluorescence de fibroblastes marqués à la rhodamine : mise en évidence du collagène de type I
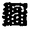 10-11 M
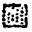 10-9 M
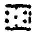 10-7 M
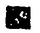 T

## Revendications

1. Conjugué peptidique **caractérisé en ce qu'**il présente la formule générale
A-X-Gly-His-Lys-Y (III),
ou
A-X-Glu-His-Lys-Y (IV)
dans laquelle
. "Lys" représente la Lysine ou un dérivé halogéné de la Lysine, ou un dérivé méthylé de la Lysine, notamment Méthyl-Lysine et Dihydrobromo-Méthyl-Lysine,
. X représente une chaine de 1 à 3 résidus Lys, éventuellement méthylés, OH, NH₂ ou une liaison
. Y représente OH ou NH₂. les acides aminés pouvant être sous la forme D, L ou DL,
ladite séquence étant conjuguée chimiquement ou physiquement avec au moins un composé A de formule gémérale (I) ou (II) ou le radical correspondant
- les acides monocarboxyliques de formule générale
HCOC-R (I)
dans laquelle R représente un radical aliphatique en C₁-C₂₀, linéaire ou ramifié, éventuellement substitué, pouvant comporter une ou plusieurs insaturations,
ainsi que les dérivés alcool ou aldéhyde ou amide des acides de formule 1 ; à la condition que si la séquence peptidique comprend uniquement Gly-His-Lys, elle n'est pas conjuguée à un seul résidu d'acide palmitique ;
- les acides dicarboxyliques de formule générale
HOOC-R₁- COOH (II)
dans laquelle R₁ représente un radical aliphatique divalent, comprenant au moins 3 atomes de carbone, de préférence 3 à 10 atomes de carbone, droit ou ramifié, notamment un radical alkyl, alkylène, alkénylène ou alkynylène, pouvant comporter une ou plusieurs insaturations éventuellement substitué, notamment par un ou plusieurs groupes amino, hydroxy, oxo ou un radical alkyl en C₁-C₃.

2. Conjugué selon la revendication 1, **caractérisé en ce que** dans la formule générale I, R peut représenter :
- un radical monoinsaturé de configuration cis ou trans, de formule générale
R₂-CH=CH-
dans laquelle R₂ peut représenter un radical alkyle linéaire ou ramifié comprenant au moins 6 atomes de carbone, de préférence 6 à 16 atomes de carbone éventuellement, substitué par un groupe amino, hydroxy ou oxo ;
- un radical aliphatique linéaire ou ramifié en C₁-C₂₀ substitué ou non, notamment un radical alkyl, alkényl ou alkynyl pouvant comporter une ou plusieurs insaturations et pouvant être substitué par un ou plusieurs radicaux choisis dans le groupe comprenant :
NH₂, OH, oxo, thiol ou un radical cyclique non aromatique comportant de 5 à 6 atomes dans le cycle dont 1 ou 2 pouvant être différents du carbone, en particulier, S, O ou N, lesdits cycles pouvant être substitués par des radicaux alkyl en C₁ à C₃, notamment méthyl.

3. Conjugué selon la revendication 2, **caractérisé en ce que** lorsque R représente une chaine aliphatique en C₁-C₂₀, elle peut être susbtituée par un cycle choisi parmi

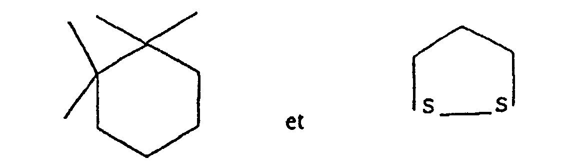

4. Conjugué selon la revendication 1, **caractérisé en ce que** dans la formule II, R₁ représente un reste alkylène en C₄-C₈ éventuellement substitué.

5. Conjugué selon l'une des revendications 1 à 4, **caractérisé en ce que** la séquence d'acides aminés est liée à l'acide de formule I ou II ou à son dérivé sous forme de sel, d'ester ou d'amide.

6. Conjugué selon l'une des revendications 1 à 4 **caractérisé en ce que** l'acide de formule générale 1 est choisi parmi l'acide acétique, l'acide DL lipoïque, l'acide dihydrolipoïque, la N-lypoyl-lysine, les acides hydroxydécénoïques et décénoïliques, l'acide rétinoïque et ses dérivés, le rétinal et le rétinol, l'acide myristique et ses dérivés, l'acide palmitique, sous forme de sels, d'esters ou amides,
et **en ce que** l'acide de formule générale II est choisi parmi l'acide adipique, l'acide α-amino adipique, l'acide pimélique, l'acide sébacique et leurs dérivés.

7. Conjugué selon l'une des revendications 1 à 6, **caractérisé en ce que** les acides de formules générales I ou II sont de préférence choisis parmi l'acide acétique et ses dérivés, l'acide α-DL-Lipoïque et ses dérivés, l'acide dihydrolipoïque, la N-Lipoyl-Lysine, l'acide adipique, l'acide α-amino-adipique, l'acide pimélique, l'acide sébacique et ses dérivés, l'acide trans-10-hydroxy-Δ2-décénoïque et l'acide trans-oxo-9-decene-2-oïque, l'acide rétinoïque et ses dérivés, le rétinol, et le rétinal, l'acide myristique et l'acide palmitique.

8. Conjugué selon l'une des revendications de 1 à 7 **caractérisé en ce qu'**il est sélectionné parmi les dérivés peptidiques suivants :
1 - A-MeLys-Lys-Lys-Gly-His-Lys-NH₂
2 - A-MeLys-Lys-Gly-His-Lys-NH₂
3 - A-MeLys-Gly-His-Lys-NH₂
4 - A-MeLys-Lys-Lys-Gly-His-Lys-OH
5 - A-MeLys-Lys-Gly-His-Lys-OH
6 - A-MeLys-Gly-His-Lys-OH
7 - A-Lys-Lys-Gly-His-Lys-NH₂
8 - A-Lys-Gly-His-Lys-NH₂
9 - A-Lys-Lys-Gly-His-Lys-OH
10- A-Lys-Gly-His-Lys-OH
11- A-Lys-Lys-Glu-His-Lys-NH₂
12- A-Lys-Glu-His-Lys-NH₂
13- A-Glu-His-Lys-NH₂
14- A-Lys-Lys-Glu-His-Lys-OH
15- A-Lys-Glu-His-Lys-OH
16- A-Glu-His-Lys-OH
"A" étant un acide de formule générale I ou II telles que définies dans les revendications de 1 à 7, ainsi que les dérivés de ces molécules sous forme de sels d'esters ou d'amides.

9. Conjugué selon l'une des revendications de 1 à 8, **caractérisé en ce qu'**il est sous forme de complexes métallopeptidiques liés physiquement ou chimiquement à un sel de Zinc.

10. Composition pharmaceutique, **caractérisée en ce qu'**elle contient au moins un conjugué selon l'une des revendications 1 à 9 avec des excipients pharmaceutiquement acceptables.

11. Composition galénique pharmaceutique, comprenant au moins un des conjugués peptidiques selon l'une des revendications 1 à 9, présentées sous forme de pommades, de crèmes dermiques, de gels, de lotions et de sprays, destinés à la médecine humaine et vétérinaire pour le traitement et la cicatrisation des plaies.

12. Composition galénique pharmaceutique, comprenant au moins un conjugué peptidique, selon l'une des revendications de 1 à 9, **caractérisée en ce que** le conjugué est associé à au moins une substance choisie parmi les antiseptiques, les antibiotiques à large spectre antimicrobien, ou les antifongiques à large spectre d'activité, destinée au traitement par voie topique et à la cicatrisation des plaies infectées.

13. Composition galénique à usage pharmaceutique et cosmétologique, comprenant au moins un des conjugués peptidiques selon les revendications de 1 à 9, présenté sous forme d'associations ou de composés, avec des molécules connues pour leurs activités anticoagulantes et phlébotoniques par voie topique, destinées au traitement préventif et curatif, des vergetures, des insuffisances veineuses (jambes lourdes), de la couperose, en applications locales, sous forme de crèmes de gels, de laits ou de sprays.

14. Composition galénique à usages pharmaceutiques et cosmétologiques, comprenant au moins un des conjugués peptidiques selon les revendications de 1 à 9 utilisés seuls, ou sous forme d'associations, de composés, ou de complexes, avec des molécules connues pour leur dérivés homologues de l'αMSH, de l'ACTH, et de la proopiomélanocortine, présentées sous forme de crèmes, de gels, de laits, de lotions ou de sprays, pour applications topiques, et destinées au traitement préventif et curatif des troubles de la mélanogénèse, à l'accélération de la mélanisation de l'épiderme, et à l'obtention d'un bronzage cutané naturel, et d'une protection totale contre les radiations solaires ultra-violettes (UVA-UVB).

15. Conjugué peptidique selon l'une des revendications 1 à 9, pour son utilisation à titre de médicament.

16. Composition cosmétologique contenant au moins un conjugué selon l'une des revendications 1 à 9 présentées sous forme de crèmes, de gels, de lotions, ou de sprays, pour applications par voie topique destinées au traitement préventif et curatif des rides du visage, du cou et des mains.

## Claims

1. Peptide conjugate, **characterized in that** it has the general formula
A-X-Gly-His-Lys-Y (III),
or
A-X-Glu-His-Lys-Y (IV)
in which
• "Lys" represents lysine or a halogenated lysine derivative, or a methylated lysine derivative, in particular methyllysine and dihydrobromomethyllysine,
• X represents a chain of 1 to 3 Lys residues, which are optionally methylated, OH, NH₂ or a bond,
• Y represents OH or NH₂,
it being possible for the amino acids to be in D, L or DL form,
the said sequence being chemically or physically conjugated with at least one compound A of general formula I or II or the corresponding radical
- monocarboxylic acids of general formula
HOOC-R (I)
in which R represents an optionally substituted, linear or branched C₁-C₂₀ aliphatic radical which can contain one or more unsaturations,
as well as the alcohol or aldehyde or amide derivatives of the acids of formula I; on condition that if the peptide sequence solely comprises Gly-His-Lys, it is not conjugated to a single palmitic acid residue;
- the dicarboxylic acids of general formula
HOOC-R₁-COOH (II)
in which R₁ represents a straight or branched divalent aliphatic radical comprising at least 3 carbon atoms, preferably 3 to 10 carbon atoms, in particular an alkyl, alkylene, alkenylene or alkynylene radical, which can contain one or more unsaturations, and which is optionally substituted, in particular with one or more amino, hydroxyl or oxo groups or a C₁-C₃ alkyl radical.

2. Conjugate according to Claim 1, **characterized in that**, in the general formula I, R can represent:
- a monounsaturated radical of cis or trans configuration, of general formula
R₂-CH=CH-
in which R₂ can represent a linear or branched alkyl radical comprising at least six carbon atoms, preferably 6 to 16 carbon atoms, optionally substituted with an amino, hydroxyl or oxo group;
- a substituted or unsubstituted, linear or branched C₁-C₂₀ aliphatic radical, in particular an alkyl, alkenyl or alkynyl radical which can contain one or more unsaturations and can be substituted with one or more radicals chosen from the group comprising:
NH₂, OH, oxo, thiol or a non-aromatic cyclic radical containing 5 or 6 carbon atoms in the ring, 1 or 2 of which can be other than carbon, in particular S, O or N, it being possible for the said rings to be substituted with C₁ to C₃ alkyl radicals, in particular methyl.

3. Conjugate according to Claim 2, **characterized in that** when R represents a C₁-C₂₀ aliphatic chain, it can be substituted with a ring chosen from

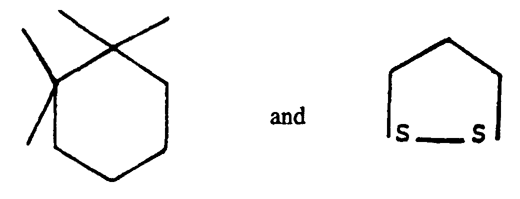

4. Conjugate according to Claim 1, **characterized in that**, in formula II, R₁ represents an optionally substituted C₄-C₈ alkylene residue.

5. Conjugate according to one of Claims 1 to 4, **characterized in that** the amino acid sequence is linked to the acid of formula I or II or to its derivative in the form of salt, ester or amide.

6. Conjugate according to one of Claims 1 to 4, **characterized in that** the acid of general formula I is chosen from acetic acid, DL-lipoic acid, dihydrolipoic acid, N-lipoyllysine, hydroxydecenoic and decenoilic acids, retinoic acid and derivatives thereof, retinal and retinol, myristic acid and derivatives thereof, and palmitic acid, in the form of salts, esters or amides,
and **in that** the acid of general formula II is chosen from adipic acid, α-aminoadipic acid, pimelic acid and sebacic acid, and derivatives thereof.

7. Conjugate according to one of Claims 1 to 6, **characterized in that** the acids of general formula I or II are preferably chosen from acetic acid and derivatives thereof, α-DL-lipoic acid and derivatives thereof, dihydrolipoic acid, N-lipoyllysine, adipic acid, α-aminoadipic acid, pimelic acid, sebacic acid and derivatives thereof, trans-10-hydroxy-Δ2-decenoic acid and trans-oxo-9-decen-2-oic acid, retinoic acid and derivatives thereof, retinol, retinal, myristic. acid and palmitic acid.

8. Conjugate according to one of Claims 1 to 7, **characterized in that** it is selected from the following peptide derivatives:
1- A-MeLys-Lys-Lys-Gly-His-Lys-NH₂
2- A-MeLys-Lys-Gly-His-Lys-NH₂
3- A-MeLys-Gly-His-Lys-NH₂
4- A-MeLys-Lys-Lys-Gly-His-Lys-OH
5- A-MeLys-Lys-Gly-His-Lys-OH
6- A-MeLys-Gly-His-Lys-OH
7- A-Lys-Lys-Gly-His-Lys-NH₂
8- A-Lys-Gly-His-Lys-NH₂
9- A-Lys-Lys-Gly-His-Lys-OH
10- A-Lys-Gly-His-Lys-OH
11- A-Lys-Lys-Glu-His-Lys-NH₂
12- A-Lys-Glu-His-Lys-NH₂
13- A-Glu-His-Lys-NH₂
14- A-Lys-Lys-Glu-His-Lys-OH
15- A-Lys-Glu-His-Lys-OH
16- A-Glu-His-Lys-OH
"A" being an acid of general formula I or II as defined in Claims 1 to 7, as well as the derivatives of these molecules in the form of salts, esters or amides.

9. Conjugate according to one of Claims 1 to 8, **characterized in that** it is in the form of metallopeptide complexes physically or chemically linked to a zinc salt.

10. Pharmaceutical composition, **characterized in that** it contains at least one conjugate according to one of Claims 1 to 9 with pharmaceutically acceptable excipients.

11. Galenic pharmaceutical composition comprising at least one of the peptide conjugates according to one of Claims 1 to 9, in the form of ointments, dermal creams, gels, lotions and sprays, intended for human and veterinary medicine for the treatment and cicatrization of wounds.

12. Galenic pharmaceutical composition comprising at least one peptide conjugate according to one of Claims 1 to 9, **characterized in that** the conjugate is combined with at least one substance chosen from antiseptics, antibiotics with a broad antimicrobial spectrum, or antifungal agents with a broad spectrum of activity, which is intended for the topical treatment and cicatrization of infected wounds.

13. Galenic composition for pharmaceutical and cosmetological use, comprising at least one of the peptide conjugates according to Claims 1 to 9, in the form of combinations or compounds, with molecules known for their anticoagulant and phlebotomic activities via the topical route, which are intended for the preventive and curative treatment of stretchmarks, venal insufficiency (tired legs) and blotches, in local application, in the form of creams, gels, milks or sprays.

14. Galenic composition for pharmaceutical and cosmetological use, comprising at least one of the peptide conjugates according to Claims 1 to 9, used alone, or in the form of combinations, of compounds, or of complexes, with molecules known for their homologous derivatives of αMSH, of ACTH, and of propiomelanocortin, which are in the form of creams, gels, milks, lotions or sprays, for topical application, and intended for the preventive and curative treatment of melanogenesis disorders, for the acceleration of epidermal melanization, for obtaining a natural skin tan, and for total protection against ultraviolet (UVA-UVB) solar radiation.

15. Peptide conjugate according to one of Claims 1 to 9, for its use as a medicinal product.

16. Cosmetological composition containing at least one conjugate according to one of Claims 1 to 9, in the form of creams, gels, lotions or sprays, for topical application, which are intended for the preventive and curative treatment of wrinkles on the face, the neck and the hands.

## Patentansprüche

1. Peptidkonjugat, **dadurch gekennzeichnet, dass** es die allgemeine Formel
A-X-Gly-His-Lys-Y (III)
oder
A-X-Glu-His-Lys-Y (IV)
aufweist, in welcher "Lys" für Lysin oder ein halogeniertes Derivat von Lysin oder ein methyliertes Derivat von Lysin, insbesondere Methyllysin und Methyllysin-dihydrobromid steht, X für eine Kette von 1 bis 3 gegebenenfalls methylierten Lys-Resten, OH, NH₂ oder eine Bindung steht, Y für OH oder NH₂ steht, wobei die Aminosäuren in der D-, L- oder DL-Form vorliegen können,
wobei die Sequenz chemisch oder physikalisch konjugiert ist mit mindestens einer Verbindung A der allgemeinen Formel (I) oder (II) oder dem entsprechenden Radikal,
- den Monocarbonsäuren der allgemeinen Formel
HOOC-R (I),
in welcher R für einen linearen oder verzweigten, gegebenenfalls substituierten aliphatischen C₁-C₂₀-Rest, der eine oder mehrere Ungesättigtheiten umfassen kann, steht,
wie auch den Alkohol- oder Aldehyd- oder Amidderivaten der Säuren der Formel I;
mit der Maßgabe, dass, wenn die Peptidsequenz einzig Gly-His-Lys umfasst, sie nicht mit einem einzelnen Palmitinsäurerest konjugiert ist;
- den Dicarbonsäuren der allgemeinen Formel
HOOC-R₁-COOH (II),
in welcher R₁ für einen zweiwertigen, geradkettigen oder verzweigten aliphatischen Rest steht, der mindestens 3 Kohlenstoffatome, vorzugsweise 3 bis 10 Kohlenstoffatome umfasst, insbesondere einen Alkyl-, Alkylen-, Alkenylen- oder Alkinylenrest, der eine oder mehrere Ungesättigtheiten umfassen kann, der gegebenenfalls, insbesondere durch eine oder mehrere Amino-, Hydroxy-, Oxogruppen oder einen C₁-C₃₋Alkylrest, substituiert ist.

2. Konjugat nach Anspruch 1, **dadurch gekennzeichnet, dass** in der allgemeinen Formel I R stehen kann für:
- einen einfach ungesättigten Rest von cis- oder trans-Konfiguration der allgemeinen Formel
R₂-CH=CH-,
in welcher R₂ für einen linearen oder verzweigten, mindestens 6 Kohlenstoffatome, vorzugsweise 6 bis 16 Kohlenstoffatome umfassenden Alkylrest, der gegebenenfalls durch eine Amino'-, Hydroxy- oder Oxogruppe substituiert ist, steht;
- einen linearen oder verzweigten, substituierten oder nicht-substituierten aliphatischen C₁-C₂₀-Rest, insbesondere einen Alkyl-, Alkenyl- oder Alkinylrest, der eine oder mehrere Ungesättigtheiten umfassen kann und substituiert sein kann durch einen oder mehrere Reste, ausgewählt aus der Gruppe umfassend:
NH₂, OH, Oxo, Thiol oder einen cyclischen, nicht aromatischen Rest, der in dem Cyclus 5 bis 6 Atome umfasst, von denen 1 oder 2 von Kohlenstoff verschieden, insbesondere S, O oder N sein können, wobei die Cyclen durch C₁- bis C₃-Alkylreste, insbesondere Methyl, substituiert sein können.

3. Konjugat nach Anspruch 2, **dadurch gekennzeichnet, dass**, wenn R für eine aliphatische C₁-C₂₀-Kette steht, sie durch einen Cyclus ausgewählt unter 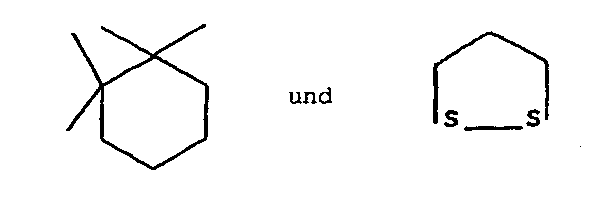 substituiert sein kann.

4. Konjugat nach Anspruch 1, **dadurch gekennzeichnet, dass** in der Formel II R₁ für einen gegebenenfalls substituierten C₄-C₈-Alkylenrest steht.

5. Konjugat nach einem der Ansprüche 1 bis 4, **dadurch gekennzeichnet, dass** die Aminosäuresequenz an die Säure der Formel I oder II oder an deren Derivat in Form von Salz, Ester.oder Amid gebunden ist.

6. Konjugat nach einem der Ansprüche 1 bis 4, **dadurch gekennzeichnet, dass** die Säure der allgemeinen Formel I ausgewählt ist unter Essigsäure, DL-Liponsäure, Dihydroliponsäure, N-Lypoyllysin, den Hydroxydecen- und Decenoylsäuren, Retinsäure und deren Derivaten, Retinal und Retinol, Myristinsäure und deren Derivate, Palmitinsäure in Form von Salzen, Estern oder Amiden,
und dass die Säure der allgemeinen Formel II unter Adipinsäure, α-Aminoadipinsäure, Pimelinsäure, Sebacinsäure und deren Derivaten ausgewählt ist.

7. Konjugat nach einem der Ansprüche 1 bis 6, **dadurch gekennzeichnet, dass** die Säuren der allgemeinen Formeln I oder II vorzugsweise unter Essigsäure und deren Derivaten, α-DL-Liponsäure und deren Derivaten, Dihydroliponsäure, N-Lipoyllysin, Adipinsäure, α-Aminoadipinsäure, Pimelinsäure, Sebacinsäure und deren Derivaten, trans-10-Hydroxy-Δ2-Decensäure und trans-Oxo-9-decen-2-säure, Retinsäure und deren Derivaten, Retinol und Retinal, Myristinsäure und Palmitinsäure ausgewählt sind.

8. Konjugat nach einem der Ansprüche 1 bis 7, **dadurch gekennzeichnet, dass** es unter den folgenden Peptidderivaten ausgewählt ist:
1 - A-MeLys-Lys-Lys-Gly-His-Lys-NH₂
2 - A-MeLys-Lys-Gly-His-Lys-NH₂
3 - A-MeLys-Gly-His-Lys-NH₂
4 - A-MeLys-Lys-Lys-Gly-His-Lys-OH
5 - A-MeLys-Lys-Gly-His-Lys-OH
6 - A-MeLys-Gly-His-Lys-OH
7 - A-Lys-Lys-Gly-His-Lys-NH₂
8 - A-Lys-Gly-His-Lys-NH₂
9 - A-Lys-Lys-Gly-His-Lys-OH
10 - A-Lys-Gly-His-Lys-OH
11 - A-Lys-Lys-Glu-His-Lys-NH₂
12 - A-Lys-Glu-His-Lys-NH₂
13 - A-Glu-His-Lys-NH₂
14 - A-Lys-Lys-Glu-His-Lys-OH
15 - A-Lys-Glu-His-Lys-OH
16 - A-Glu-His-Lys-OH
wobei "A" eine Säure der allgemeinen Formel I oder II, wie in den Ansprüchen 1 bis 7 definiert, wie auch die Derivate dieser Moleküle in Form von Salzen, Estern oder Amiden ist.

9. Konjugat nach einem der Ansprüche 1 bis 8, **dadurch gekennzeichnet, dass** es in Form von Metall-Peptid-Komplexen, die physikalisch oder chemisch an ein Zinksalz gebunden sind, vorliegt.

10. Pharmazeutische Zusammensetzung, **dadurch gekennzeichnet, dass** sie mindestens ein Konjugat nach einem der Ansprüche 1 bis 9 mit pharmazeutisch akzeptablen Trägersubstanzen enthält.

11. Galenische pharmazeutische Zusammensetzung, umfassend mindestens eines der Peptidkonjugate nach einem der Ansprüche 1 bis 9, dargereicht in Form von Pomaden, Hautcremes, Gelen, Lotionen und Sprays, die für die Human- und Veterinärmedizin für die Behandlung und die Vernarbung von Wunden bestimmt sind.

12. Galenische pharmazeutische Zusammensetzung, umfassend mindestens ein Peptidkonjugat nach einem der Ansprüche 1 bis 9, **dadurch gekennzeichnet, dass** das Konjugat mit mindestens einer Substanz, die unter den Antiseptika, den Antibiotika mit breitem antimikrobiellem Spektrum oder den Antimykotika mit breitem Wirkungsspektrum ausgewählt ist, kombiniert ist, die für die Behandlung auf topischem Wege und die Vernarbung von infizierten Wunden bestimmt ist.

13. Galenische Zusammensetzung zur pharmazeutischen und kosmetischen Verwendung, umfassend mindestens eines der Peptidkonjugate nach den Ansprüchen 1 bis 9, dargereicht in Form von Kombinationen oder Zusammensetzungen mit Molekülen, die für ihre gerinnungshemmenden und phlebotonen Eigenschaften auf topischem Wege bekannt sind, die für die präventive und heilende Behandlung von Striemen, Veneninsuffizienz (schweren Beinen), Kupferausschlag bei lokaler Anwendung bestimmt sind, in Form von Cremes, Gelen, Milchpräparaten oder Sprays.

14. Galenische Zusammensetzung für pharmazeutische und kosmetische Verwendungen, umfassend mindestens eines der Peptidkonjugate nach den Ansprüchen 1 bis 9, die allein verwendet werden oder in Form von Kombinationen, Zusammensetzungen oder Komplexen mit Molekülen, die für ihre zu αMSH, ACTH und Proopiomelanocortin homologen Derivate bekannt sind, dargereicht in Form von Cremes, Gelen, Milchpräparaten, Lotionen oder Sprays, für topische Anwendungen und die für die präventive und heilende Behandlung von Störungen der Melanogenese, die Beschleunigung der Melanisation der Epidermis und die Erzielung einer natürlichen Hautbräunung und eines vollständigen Schutzes gegen die ultravioletten Sonnenstrahlen (UVA-UVB) bestimmt sind.

15. Peptidkonjugat nach einem der Ansprüche 1 bis 9 für dessen Verwendung als Arzneimittel.

16. Kosmetische Zusammensetzung, umfassend mindestens ein Konjugat nach einem der Ansprüche 1 bis 9, dargereicht in Form von Cremes, Gelen, Lotionen oder Sprays für Anwendungen auf topischem Wege, die für die präventive und heilende Behandlung der Falten des Gesichts, des Halses und der Hände bestimmt sind.
